Europäisches Patentamt

European Patent Office

Office européen des brevets

(19)

(11) Publication number: **0 679 403 A1**

## (12) EUROPEAN PATENT APPLICATION
published in accordance with Art. 158(3) EPC

(21) Application number: **95900920.0**

(22) Date of filing: **18.11.94**

(86) International application number:
**PCT/JP94/01954**

(87) International publication number:
**WO 95/13826 (26.05.95 95/22)**

(51) Int. Cl.6: **A61K 38/06**, A61K 38/07,
//C07K5/09, C07K5/11

(30) Priority: **18.11.93 JP 289037/93**
**25.10.94 JP 260044/94**

(43) Date of publication of application:
**02.11.95 Bulletin 95/44**

(84) Designated Contracting States:
**AT BE CH DE DK ES FR GB GR IE IT LI LU MC NL PT SE**

(71) Applicant: **MEIJI MILK PRODUCTS COMPANY LIMITED**
**3-6, Kyobashi 2-chome**
**Chuo-ku**
**Tokyo 104 (JP)**

(72) Inventor: **KINO, Kohsuke, Meiji Institute of Health Science**
**540, Naruda**
**Odawara-shi**
**Kanagawa 250 (JP)**

(74) Representative: **Weisert, Annekäte, Dipl.-Ing. Dr.-Ing.**
**Patentanwälte**
**Kraus Weisert & Partner**
**Thomas-Wimmer-Ring 15**
**D-80539 München (DE)**

(54) **PREVENTIVE AND REMEDY FOR ALLERGIC DISEASE.**

(57) A preventive and remedy for allergic diseases containing a peptide having the amino acid sequence represented by Arg-Gly-Asp as the active ingredient. It is useful for preventing and treating various symptoms of allergic diseases including allergic conjunctivitis, rhinitis, dermatitis, and asthma.

Fig. 3

Technical Field:

The present invention relates to prophylactic and therapeutic agents for allergic diseases, and more particularly to prophylactic and therapeutic agents for allergic diseases which prevent various allergy symptoms by inhibiting the degranulation phenomenon of chemical transmitters such as histamine present in sensitized cells.

Background Art:

The number of patients suffering from allergic diseases has increased year by year. In Japan, according to the investigation by the Ministry of Health and Welfare in 1992, one third of the population in Japan showed some allergy symptoms. It is said that the changes in the living environment including the housing environment, which increase mite allergens, increase allergic patients. Others such as changes in diet, stress, and air pollution also contribute to the increase in allergies.

Although allergic diseases have a wide variety of symptoms, most of them are categorized as type I allergies (also called immediate-type allergies) which include bronchial asthma, pollinosis, atopic dermatitis, allergic enterogastritis, and food allergies.

Antigens that cause an allergic reaction are called allergens. Major allergens (which may be referred to in this description as antigens) include foods such as milk, eggs, and soybeans; pollens of cedar, Japanese cypress, white birch, and hogweed; and mites in house dust that inhabit the living environment. These allergens frequently induce allergic reactions.

The onset mechanism of type I allergies is roughly divided into the following three stages:

Stage I: Various antigens which have entered into the human body are encapsulated by antigen-presenting cells such as macrophages, in which the antigens are degraded into peptides. Some parts of the peptides (the epitopes recognized by T cells) form complexes with their own MHC proteins, thereby transmitting their antigenity to activate T cells. The activated T cells accelerate differentiation and progression of B cells, and induce production of IgE antibodies which are specific to antigens. The IgE antibodies thus produced are bound to IgE receptors on the surface of mast cells which are target cells present in the skin or in the mucosal tissue, or on the surface of basophils in peripheral blood, to establish a sensitized state.

Stage II: Antigens which have invaded the human body bind themselves to 2 or more molecules of IgE antibodies on the surface of sensitized cells and induce cross-linking of the antibodies through the antigens. This cross-linking phenomenon on the cell surface triggers degranulation and release of chemical transmitters such as histamine and leuckotriene.

Stage III: The thus-released chemical transmitters cause a contraction of smooth muscle and an increase in capillary permeability, producing so-called allergic symptoms.

Type I allergies are prevented and treated by the administration of anti-allergy drugs, immunotherapy, and diet therapy. Many anti-allergy drugs have their working point in stage II, and they are grouped into two groups. The first is acidic anti-allergy drugs which are said to inhibit the release of chemical transmitters by stabilizing cell-surface membrane. The second is basic anti-allergy drugs which possess an anti-histamine action working in stage III. Immunotherapy has long history, but is no longer popular due to a number of drawbacks, including adverse side effects such as anaphylaxis which is caused by repeated subcutaneous injections of causal allergens to the allergic patient in small amounts, frequent visits to the clinic, and a long period of treatment which are a burden on the patient. The diet therapy prevents the onset of allergy by eliminating causal allergens from the daily dietary life. However, allergens are not easily identified, and in addition, cooking becomes considerably complicated.

Extracellular matrices such as fibronectin, collagen, and laminin are indispensible for the formation of connective tissues in the course of developing tissues and organs. These extracellular matrices, which are synthesized by fibroblasts, epithelial cells, endothelial cells, and cartilage cells, associate to form a fibrous and network structure on the outer surface of the cells, whose structure further agglomerates to finally form blood vessel basal membranes and connective tissues. Most of the constituents of these extracellular matrices have afinity to the cells and form a tissue starting from the cells as the core.

Among these extracellular matrices, fibronectin has a very high afinity to the cells. From the results of the analyses of the relation between amino acid sequence and cellular adhesion activities, it was discovered that the high afinity of fibronectin to cells is a result of the binding of 4 amino acids of Arg-Gly-Asp-Ser in a fibronectin molecule (RGDS; based on single-character representation of the amino acids, in which three amino acids of RGD are essential) and a fibronectin receptor (FNR) present on the cell-surface membrane (M.D. Pierschbacher, E. Ruoslahti, Nature 309: 30-33, 1984). In other words, a peptide containing an RGD sequence or an RGDS sequence (hereinafter referred to as RGD(RGDS)) inhibits the interaction between fibronectin and cells.

Recently, receptors to extracellular matrices such as fibronectin, laminin, and collagen have been identified one after another in cells of the immune system. It suggests that the interaction between these extracellular matrices and the receptors is important in the differentiation of T cells, homing of lymphocytes, etc. These receptors are collectively called an integrin super family because they possess a common subunit structure. VLA's (very late antigens) found in human T cells are also categorized as the integrin super family. VLA-5 is immunologically identical to FNR, and is inhibited to bind fibronectin in the presence of an RGD peptide (Y. Takada et al., Nature 326: 607-609, 1987). FNR(VLA-5) is expressed on the cell-surface membrane of cultured mast cells of rat. When it is bound to its ligand fibronectin, a histamine releasing reaction via antigen - IgE antibody - IgE receptor in the type I allergy reaction is amplified (M.M. Hamawy et al., J. Immunol. 149: 615-621, 1992).

Under the present situation, however, the physiological role of FNR (VLA-5) on mast cells, bisophils, or T cells is not known in detail.

An object of the present invention is to provide a prophylactic and therapeutic agent for allergic diseases based on a new working mechanism different from conventional mechanisms, which works in the second stage of the above-mentioned allergic reaction, i.e., release of chemical transmitters such as histamine is inhibited by using the phenomenon of antigens on sensitized cell surfaces.

Disclosure of the Invention:

The present inventors studied whether FNR's (VLA-5) which have been found on the cell-surface membrane of cultured mast cells of rats exist also on the cell-surface membrane of bosophils in human peripheral blood, and they were successful in finding the receptors thereon. Further, they found that a strong histamine release reaction occurs when FNR's (VLA-5) are cross-linked on the cell-surface of the membrane by the use of anti-FNR antibodies, This outcome substantiates that FNR's (VLA-5) function directly or indirectly in cooperation with the histamine release reaction of bosophils. In this connection, it is noteworthy that histamine release reactions caused in bosophils of patients suffering from pollinosis of Japanese cedars due to Cryj I (WO93/01213), a major allergen of pollens of Japanese cedar, was strongly inhibited by RGD(RGDS). Moreover, histamine release reactions induced when crudely extracted antigens of mite are added to bosophils of patients suffering from mite allergy were also strongly inhibited when RGD(RGDS) peptide is added. Furthermore, when RGD or RGDS peptides were added to a certain experimental system widely applicable to various antigens in which histamine is urged to be released by the cross-linking of IgE bound to IgE receptors of bosophils of a patient using anti-IgE antibodies, the release of histamine was strongly inhibited. Namely, the present inventors found that RGD(RGDS) peptides are useful as anti-allergy drugs against allergic diseases originated from various antigens from the fact that RGD(RGDS) peptides strongly inhibit the release reaction of histamine from mast cells or bosophils in blood of allergic patients who are sensitive to various allergens such as Cryj I or mite allergens, leading to completion of the invention.

Accordingly, the present invention provides a prophylactic and therapeutic agent for allergic diseases which comprises, as its effective component, a peptide containing an amino acid sequence represented by RGD(RGDS) or a salt thereof.

The present invention also provides a pharmaceutical composition for preventing and treating allergic diseases which comprises a peptide containing an amino acid sequence represented by RGD(RGDS) or a salt thereof and a pharmaceutically acceptable carrier.

The present invention further provides a method of preventing and treating allergic diseases characterized by administering an effective amount of a peptide containing an amino acid sequence represented by RGD(RGDS) or a salt thereof.

The present invention further provides a use of a peptide containing an amino acid sequence represented by RGD(RGDS) or a salt thereof for the purpose of preventing and treating allergic diseases.

Brief Description of the Drawings:

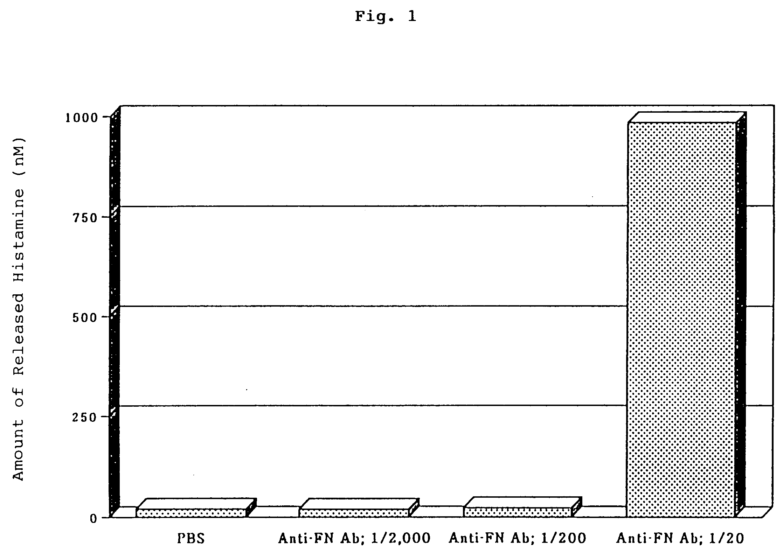

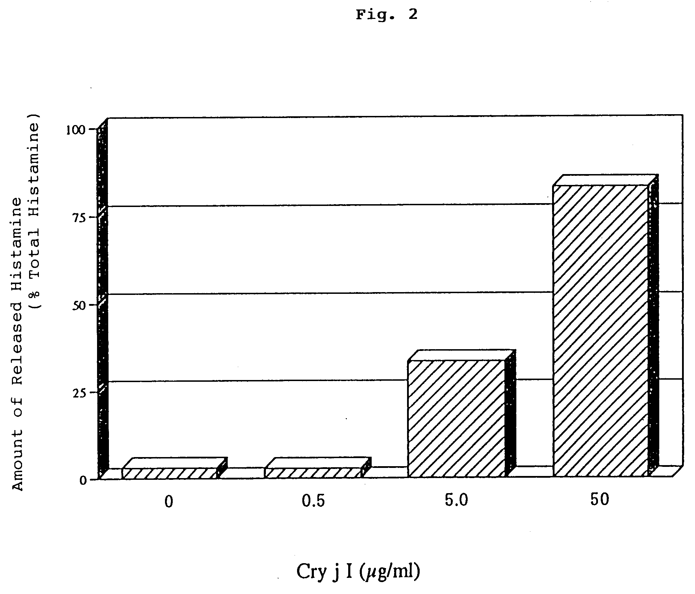

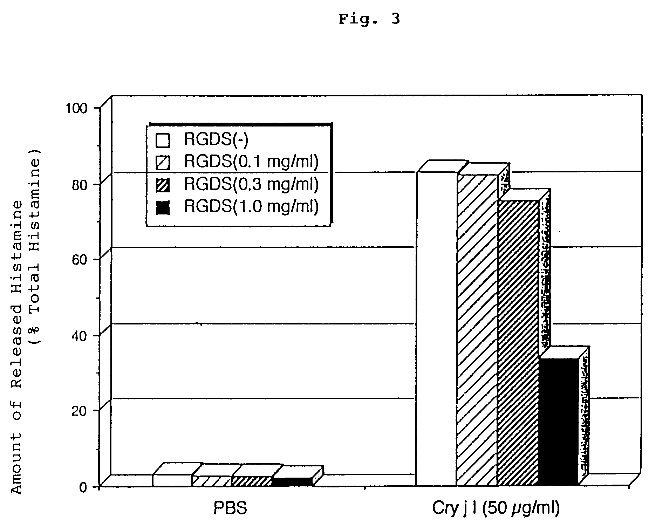

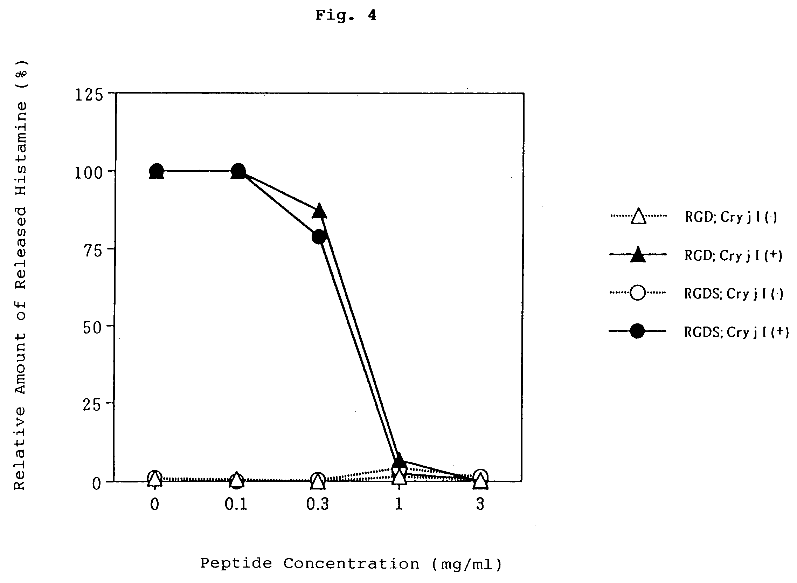

Fig. 1 shows a histamine release reaction from human bosophils by addition of anti-fibronectin antibodies. Fig. 2 shows a histamine release reaction from bosophils of patients suffering from pollinosis of Japanese cedar by addition of Cryj I (WO93/01213). Fig. 3 shows an inhibiting action of RGDS against the histamine release from bosophils of patients suffering from pollinosis of Japanese cedar by addition of Cryj I, the pollen allergen of Japanese cedar. Fig. 4 shows a comparison between inhibiting actions of RGDS

3

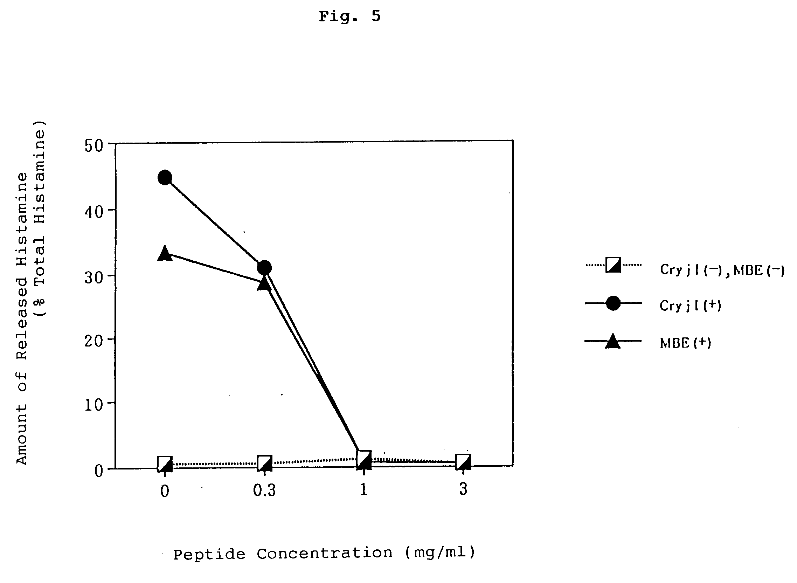

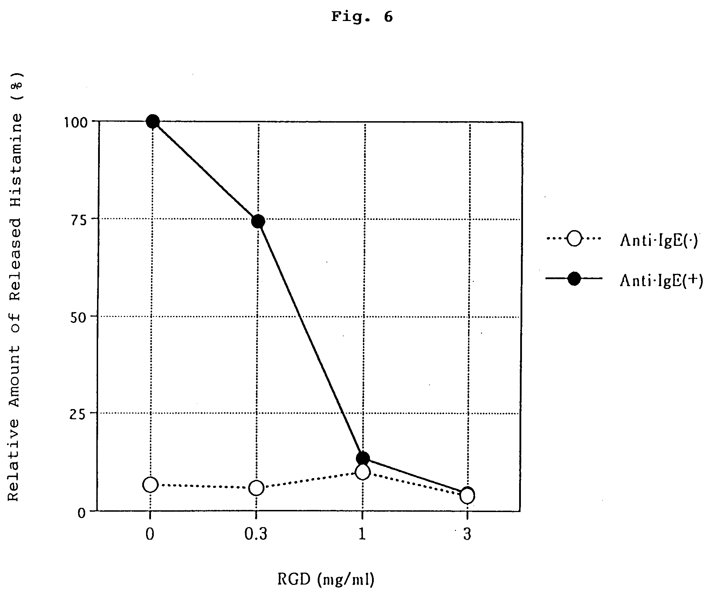

peptides and RGD peptides against the histamine release from bosophils of patients suffering from pollinosis of Japanese cedar by addition of Cryj I, the pollen allergen of Japanese cedar. Fig. 5 shows inhibiting actions of RGD peptides against the histamine release by addition of each allergen, Cryj I or MBE (a mite body extract allergen), from basophilic cells of patients who presented positive results to both the cedar pollen allergen and mite allergen. Fig. 6 shows the inhibiting effects of RGD peptide against histamine release caused by anti-IgE antibody.

Best Mode for Carrying Out the Invention:

The peptide of the present invention containing an amino acid sequence represented by RGD(RGDS) is not particularly limited as long as it has an amino acid sequence represented by RGD. However, in view that the object of the present invention is to prevent and treat allergic diseases, it is preferred that it does not contain other amino acid sequences which have other unnecessary actions. From this point of view, RGD(RGDS) and their salts are preferable peptides. Examples of the salts include those of organic and inorganic acids such as acetic acid, hydrochloric acid, sulfuric acid, and nitric acid; alkali metal salts such as sodium and potassium; and alkaline earth metal salts such as calcium and magnesium. The N-terminal and/or C-terminal of these peptides may be protected with an alkanoyl group, alkyl group, amino group, etc. The amino acids that constitute these peptides may be in the L-, D-, or DL- form.

The RGD(RGDS) peptides of the present invention can be prepared according to a routine process for synthesizing peptides such as a solid phase peptide synthesis method and a liquid phase peptide synthesis method.

The RGD(RGDS) peptides of the present invention work on various causal allergens of type I allergies, and strongly inhibit the histamine release reaction caused by the respective allergens. Therefore, they can be used for the prevention and treatment of various symptoms caused from type I allergic reactions such as allergic conjunctivitis, rhinitis, dermatitis, and asthma. Especially, they are useful for the prevention and treatment of allergic diseases caused from pollen or mite allergens.

The manner of administering the RGD(RGDS) peptides of the present invention is not particularly limited. These may be administered in a therapeutical manner to patients who currently develop allergy symptoms or may be administered in a preventive manner in advance of developing allergy symptoms.

The RGD(RGDS) peptides of the present invention can be administered orally, parenterally, or in any other forms. The form of compositions for oral use may be tablets, granules, capsules, syrups, and lozenges. The form of parenteral compositions may be injections, nasal drops, eye drops, and percutaneous agents. When these compositions are prepared, carriers which are ordinarily used in the manufacture of pharmaceuticals, such as vehicles, binders, disintegrants, lubricant, and solution adjuvants, may be used.

The dose of the RGD(RGDS) peptides of the present invention differs according to the stage of the disease, body weight, age, and symptoms. Preferably, the dose is 1 to 1000 mg/kg/day as the amount of peptide.

Examples:

The present invention will hereinafter be described in detail by way of examples, which should not be construed as limiting the present invention.

Example 1:

Purification of Cryj I:

Pollens were collected from male flowers blossomed on sprays of Japanese cedar trees cut in February in Shizuoka and Kanagawa prefectures. They were frozen at -70°C. Cryj I was purified according to a Yasueda's method (Allergy, 29: 763-772, 1980), i.e., cedar pollens were applied on ion-exchange column chromatography using DE52, CM52 (Whatman) gels.

Example 2:

FNR(VLA-5) on the cell-surface membrane of human basophils:

RBL-2H3 cells are cultured cells of rats, and are widely used in the field of allergy research as models of mast cells and basophils in vitro. It has already been confirmed that FNR(VLA-5) is present on the cell-surface membrane of RBL-2H3 (Katsuhiko YASUDA, et al., Proceedings of the Academic Assembly of Japan Immunological Society 21: 530, 1991; M.M. Hamawy et al., J. Immunol. 149: 615-621, 1992; Katsuhiko YASUDA, et al., Proceedings of the Academic Assembly of Japan Immunological Society 22: 346, 1992).

However, it has not yet been confirmed whether FNR(VLA-5) is present on basophils of human peripheral blood. The present inventors studied the possibility of such a presence using a histamine release test.

Venous blood samples were collected in the presence of heparin from healthy humans. Each blood sample was diluted with the same amount of a buffer solution for release tests, and the diluted blood was placed in a 96-well V-bottom plate in portions of 50 $\mu$l each. 100 $\mu$l of phosphate-buffered saline (PBS, pH 7.2) was added. Further, a sheep anti-human fibronectin antibody (the Binding Site LTD) solution was added in portions of 50 $\mu$l each so that final dilution ratios of 1/20, 1/200, and 1/2,000 were obtained. The reaction was allowed to proceed at 37°C for 30 minutes. As a control, a sample in which 50 $\mu$l of PBS was contained in place of sheep anti-human fibronectin antibody (anti-FN Ab) was provided which served as a negative control. Fibronectin was not added to this experimental system because plasma contains it in an amount of 30 mg/dl (previously called as insoluble cryoglobulin), and therefore the addition was not necessary. After completion of the reaction, the system was subjected to a centrifugal separation at 1,500 rpm for 5 minutes. The histamine concentration in 100 $\mu$l of the supernatant was measured with a histamine kit "Eiken" manufactured by Eiken Kagaku K.K. The results are shown in Fig. 1.

As is apparent from Fig. 1, in the negative control and in samples of low concentrations of antibody (anti-FN Ab: 1/2,000, Anti-FN Ab: 1/200 dilutions), histamine release was scarcely observed. By contrast, at the highest concentration of antibody, at a dilution of 1/20 (Anti-FN Ab: 1/20), significant level of histamine release was observed. From these results, it is considered that FNR(VLA-5) is present on human basophils, which are the cells that contain histamine in the greatest amount among blood cells, like rat cultured cells, and that plasma fibronectin is bound to basophils through its receptor, and that histamine is released as a result of the cross-linking of the resulting fibronectin-FNR(VLA-5) complexes on cell surfaces by the aid of sheep anti-human fibronectin antibody.

This fact reflects a newly discovered mechanism that histamine release is induced as a result of the formation of cross-linkage of FNR(VLA-5). This mechanism is apart from the old understanding that the histamine release reaction in mast cells and basophils is caused as a result of the formation of cross-linkage by complexes of allergens-IgE-IgE receptors or the formation of cross-linkage caused as a result of the addition of anti-IgE receptor antibodies.

Example 3:

Determination of optimum concentration in histamine release reaction by Cryj I:

Causal allergens of allergy patients are identified by adding allergens to patient's blood and measuring the amount of histamine released. This method permits a very sensitive approach for identifying allergens specific to the patient. However, since allergens which are added have an optimum concentration, the amount of released histamine cannot always be detected if its level is too high or too low. Accordingly, using blood from cedar-pollinosis patients, the optimum concentration of Cryj I in histamine release reaction was investigated. Venous blood was collected from cedar-pollinosis patients in the presence of heparin, and the amount of histamine reLeased in the presence of Cryj I in a variety of concentrations was measured in a manner similar to that described in Example 2. The results are shown in Fig. 2.

As is apparent from Fig. 2, when the amount of addition of Cryj I was made greater, the amount of released histamine increased. At the concentration of 50 $\mu$g/ml, 83% of total histamine was released. Therefore, the concentration of Cryj I was set to 50 $\mu$g/ml, and a histamine release inhibition test was conducted using an RGDS peptide.

Example 4:

Inhibition by RGDS peptide in histamine release reaction using Cryj I:

Venous blood sample were collected in the presence of heparin from cedar-pollinosis patients. Each blood sample was diluted with the same amount of a buffer solution for release tests, and the diluted blood was placed in a 96-well V-bottom plate in portions of 50 $\mu$l each. 100 $\mu$l of a solution of RGDS peptide (product of Peptide Laboratories) in PBS was added go that final concentrations of 0.1, 0.3, and 1.0 mg/ml were obtained. As a control, a system solely consisting of PBS was also prepared. 50 $\mu$l of Cryj I was added to each well at a the final concentration of 50 $\mu$g/ml was obtained. As a control, a system solely consisting of 50 $\mu$l of PBS was also prepared.

The reaction was allowed to proceed at 37°C for 30 minutes. After completion of the reaction, the amount of released histamine in 100 $\mu$l of a supernatant was measured in a manner similar to that described in Example 2. The results are shown in Fig. 3.

As is apparent from Fig. 3, when the concentration of RGDS peptide became greater, the amount of released histamine in the presence of Cryj I was suppressed. When the concentration of RGDS peptide was 1.0 $\mu$g/ml, the inhibition rate was approximately 60% in comparison to the rate in the peptide-free system. On the other hand, when Cryj I was not present, histamine release was scarcely observed over the concentration range of RGDS peptide from 0.1 mg/ml to 1.0 mg/ml.

Additionally, in order to confirm the validity of RGDS peptide, a test of histamine release inhibition by RGDS peptide was carried out using venous blood samples from 17 subjects consisting of 14 cedar-pollinosis patients and 3 controls of healthy humans. A negative control was prepared by using a synthetic peptide composed of an Ala-Asn-Gln-Asn-Thr-Lys-Thr-Ala-Lys residue, which is a partial peptide of mite allergen Der p II (amino acid sequence Nos. 43-51) instead of RGDS peptide. The reaction conditions were as described above.

As a result, as shown in Table 1, 55.20% of the total amount of blood histamine was released by the addition of Cryj I in the group of cedar-pollinosis patients, whereas 2.36% was released in the group of healthy subjects, revealing that the patient group specifically responded to Cryj I. When RGDS peptide was added to this experimental system, the amount of released histamine decreased to 2.91%. This result indicates that the release of histamine was approximately quantitatively inhibited by RGDS peptide (it is interpreted that the suppression was significant at a significance level of not higher than 0.1%). When a partial peptide of Der p II which is irrelevant to the present experimental system was used in place of RGDS peptide, the histamine release was 59.60%, which was not significantly distinguished from the group which did not contain RGDS peptide. In the group of healthy subjects, statistically significant difference was not observed regarding the histamine release in all the reactions. From the above results, it was proved that RGDS peptide significantly inhibited the histamine release reaction caused by Cryj I allergen.

Table 1 Inhibition by RGDS Peptide of Histamine Release Reaction Caused by Cedar Pollen Allergen, Cryj I, obtained from Blood of Cedar-Pollinosis Patients

| Blood from cedar-pollinosis patients or healthy humans | Release of Histamine (%total histamine) | | | |
|---|---|---|---|---|
| | Cryj I-free | | Cryj I added | |
| | RGDS added | RGDS free | Control peptide added (note 1) | RGDS added |
| Blood from cedar-pollinosis (14 subjects) | $2.52 \pm 1.46^{***}$ | $55.20 \pm 27.37$ (note 2) | $59.60 \pm 27.60^{n.s.}$ | $2.92 \pm 2.04^{***}$ |
| Blood from healthy humans (3 subjects) | $2.05 \pm 0.79^{n.s.}$ | $2.36 \pm 1.38$ (note 3) | $1.77 \pm 0.91^{n.s.}$ | $2.51 \pm 1.41^{n.s.}$ |

(note 1): As a negative control regarding RGDS peptide, a synthetic peptide composed of 9 residues of Der p II allergen (43-51) was added.

(note 2), (note 3): Testing of significance based on a Dunnett-type multiple comparison using the "Cryj I added" + "RGDS free" groups as a basis of comparison between blood samples from cedar-pollinosis and from healthy humans.

(***; $p<0.1\%$, n.s.; not significant)

Example 5:

Comparison of RGDS peptide and RDS peptide with respect to their activities:

In a manner similar to that described in Example 4, venous blood samples from cedar pollinosis patients were used and inhibitory actions of RGDS peptide and RDS peptide against histamine release reaction were compared. The results are shown in Fig. 4.

As is apparent from Fig. 4, under the co-existence of Cryj I, RGDS peptide and RGD peptide both exhibited almost the same level of inhibitory action against the release of histamine, thus both were effective. When Cry j I was not present, histamine release was scarcely observed in both cases of the use of RGDS peptide and RGD peptide in amounts from 0.1 mg/ml to 3.0 mg/ml.

Example 6:

Inhibition of histamine release reaction by RGD peptide in blood from mite allergy patients:

Venous blood samples were collected in the presence of heparin from patients who were RAST positive with a value 4 or more against mite and cedar allergens. Using the obtained venous blood samples in a manner similar to that described in Example 4, inhibitory effects of RGDS peptide against histamine release were investigated in the presence of 50 $\mu$g/ml of Cryj I or 0.1 $\mu$g/ml of a PBS extract solution of mite bodies (MBE) (an optimum MBE concentration was determined as described in Example 3) regarding respective allergens. In this connection, the MBE which was used was a supernatant obtained by homogenizing mite bodies in a PBS solution and effecting a centrifugal separation at 12,000 x g for 20 minutes. The results are shown in Fig. 5.

As is apparent from Fig. 5, under the conditions where Cryj I or MBE was present and RGD peptide was not added, histamine release was caused by Cryj I or MBE. By contrast, when RGD peptide was added, both Cryj I and MBE suppressed histamine release depending on the concentration of RGD peptide.

Accordingly, RGD peptide is effective in the treatment and prevention of allergy diseases of patients with cedar pollinosis or mite allergies.

Example 7:

Inhibitory effects of RGD peptide against a histamine release reaction caused by anti-IgE antibody:

A histamine release reaction with an anti-IgE antibody is a reaction in which histamine is released as a result of cross-linking of IgE antibodies of the patient bound to IgE receptors by the mediation of anti-IgE antibodies on the surface of basophils. Since this reaction is caused by the coagulation of IgE receptors as a result of the formation of anti-IgE antibody - IgE - IgE receptor in a histamine release mechanism similar to that in the presence of various allergens, it is widely used as an experimental system regarding histamine release reactions which are commonly applicable to various allergens (T. Ishizaka et al., J. Immunol. 130: 2357-2362, 1983; K.E. Barrett et al., J. Immunol. 137: 2001-2008, 1986; "Current Protocols in Immunology" edited by J.E. Coligan at al., Suppl. 1., 7.24.3, 7.25.3 (1991)).

Venous blood samples were collected in the presence of heparin from 5 humans including patients suffering from pollinosis and healthy subjects. The blood samples were placed in a 96-well V-bottom plate, and the procedure of Example 6 was repeated to investigate the inhibitory effect of RGD peptide in a histamine release reaction caused by an anti-IgE antibody (mouse anti-human IgE monoclonal antibody; product of Pierce) (2.5 $\mu$g/ml, an optimum anti-IgE antibody concentration was determined as described in Example 3). The results are shown in Fig. 6. Fig. 6 represents the result of one case among 5. However, the remainder 4 cases presented almost the same results. The amount of histamine released due to the presence of the anti-IgE antibody in the absence of RGD peptide was taken as 100%. As the RGD concentration increased from 0.3 to 1 and further to 3 mg/ml, the amount of histamine release was inhibited. From this result, it was proved that RGD peptide is effective in inhibiting the histamine release reactions caused by RGD peptide with respect to various allergens.

Industrial Applicability:

The RGD(RGDS) peptide of the present invention concentration-dependently inhibits the release of histamine from mast cells or basophils of allergy patients who are sensitive against a variety of allergens, and therefore, the peptide is useful as an anti-allergy agent.

**Claims**

1. A prophylactic and therapeutic agent for an allergic disease which comprises, as its effective component, a peptide containing an amino acid sequence represented by Arg-Gly-Asp or a salt thereof.

2. The prophylactic and therapeutic agent according to Claim 1, wherein the amino acid sequence of the peptide is composed of Arg-Gly-Asp or Arg-Gly-Asp-Ser.

3. The prophylactic and therapeutic agent according to Claim 1, wherein the allergic disease is a disease caused by a type-I allergy reaction.

4. The prophylactic and therapeutic agent according to Claim 1, wherein the allergy disease is a disease caused by a type-I allergy reaction in which the allergen is pollens or mites.

5. A pharmaceutical composition for preventing and treating allergic diseases which comprises a peptide containing an amino acid sequence represented by Arg-Gly-Asp or a salt thereof and a pharmaceutically acceptable carrier.

6. The composition according to Claim 5, wherein the amino acid sequence of the peptide is composed of Arg-Gly-Asp or Arg-Gly-Asp-Ser.

7. A method of preventing and treating allergic diseases characterized by administering an effective amount of a peptide containing on amino acid sequence represented by Arg-Gly-Asp or a salt thereof.

8. The method according to Claim 7, wherein the amino acid sequence of the peptide is composed of Arg-Gly-Asp or Arg-Gly-Asp-Ser.

9. A use of a peptide containing an amino acid sequence represented by Arg-Gly-Asp or Arg-Gly-Asp-Ser or a salt thereof for the purpose of preventing and treating allergic diseases.

10. The use according to Claim 9, wherein the amino acid sequence of the peptide is composed of Arg-Gly-Asp or Arg-Gly-Asp-Ser.

Fig. 1

EP 0 679 403 A1

Fig. 2

Cry j I ($\mu$g/ml)

EP 0 679 403 A1

Fig. 3

EP 0 679 403 A1

Fig. 4

Fig. 5

Fig. 6

Relative Amount of Released Histamine (%)

RGD (mg/ml)

····O···· Anti·IgE(·)

──●── Anti·IgE(+)

EP 0 679 403 A1

International application No.

PCT/JP94/01954

**A. CLASSIFICATION OF SUBJECT MATTER**

Int. Cl$^6$ A61K38/06, 38/07//C07K5/09, 5/11·

According to International Patent Classification (IPC) or to both national classification and IPC

**B. FIELDS SEARCHED**

Minimum documentation searched (classification system followed by classification symbols)

Int. Cl$^6$ A61K38/06, 38/07

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| A | J. Biol Chem. Vol.264 No. 34 (1989), P.20502-20508 J. Martinez, et al., "Transglutaminase-mediated cross-linking of fibrinogen by human unbilical vein endothelial cells" | 1-6, 9, 10 |
| A | Chemical Abstracts Vol. 112(1990) abstracts number 69492 | 1-6, 9, 10 |

☐ Further documents are listed in the continuation of Box C.    ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| February 6, 1995 (06. 02. 95) | February 28, 1995 (28. 02. 95) |

| Name and mailing address of the ISA/ | Authorized officer |
|---|---|
| Japanese Patent Office | |
| Facsimile No. | Telephone No. |

Form PCT/ISA/210 (second sheet) (July 1992)

**Box I**    Observations where certain claims were found unsearchable (Continuation of item 1 of first sheet)

This international search report has not been established in respect of certain claims under Article 17(2)(a) for the following reasons:

1. ☒   Claims Nos.: 7, 8
   because they relate to subject matter not required to be searched by this Authority, namely:
   Claims 7 and 8 pertain to methods for treatment of the human or animal body by therapy, and thus relates to a subject matter which this International Searching Authority is not required, under the provisions of Article 17(2)(a)(i) of the PCT and Rule 39.1(iv) of the Regulations under the PCT, to search.

2. ☐   Claims Nos.:
   because they relate to parts of the international application that do not comply with the prescribed requirements to such an extent that no meaningful international search can be carried out, specifically:

3. ☐   Claims Nos.:
   because they are dependent claims and are not drafted in accordance with the second and third sentences of Rule 6.4(a).

**Box II**    Observations where unity of invention is lacking (Continuation of item 2 of first sheet)

This International Searching Authority found multiple inventions in this international application, as follows:

1. ☐   As all required additional search fees were timely paid by the applicant, this international search report covers all searchable claims.

2. ☐   As all searchable claims could be searched without effort justifying an additional fee, this Authority did not invite payment of any additional fee.

3. ☐   As only some of the required additional search fees were timely paid by the applicant, this international search report covers only those claims for which fees were paid, specifically claims Nos.:

4. ☐   No required additional search fees were timely paid by the applicant. Consequently, this international search report is restricted to the invention first mentioned in the claims; it is covered by claims Nos.:

**Remark on Protest**    ☐   The additional search fees were accompanied by the applicant's protest.

                               ☐   No protest accompanied the payment of additional search fees.